# EUROPEAN PATENT APPLICATION

(11) **EP 4 664 469 A1**
(43) Date of publication of application: **17.12.2025**
(21) Application number: 24181291.6
(22) Date of filing: 11.06.2024
(51) Int. Cl.: G16H 20/30, G16H 50/70

(54) **MACHINE LEARNING MODEL AS REWARD FUNCTION FOR REINFORCEMENT LEARNING ALGORITHM FOR SURGICAL PLANNING**

(71) Applicant: Siemens Healthineers AG, 91301 Forchheim (DE)
(72) Inventor: Audigier, Chloé, 3006 Bern (CH); Kellnberger, Stephan, 85604 Zorneding (DE); Meister, Felix, 91054 Erlangen (DE)
(74) Representative: Siemens Healthineers Patent Attorneys

(57) **Abstract**

The disclosure relates to fine-tuning a pre-trained reinforcement learning algorithm, which facilitates a determination of surgical planning for treating a disease associated with an anatomical target region. The pre-trained reinforcement learning algorithm is fine-tuned using a repetitively updated machine learning model. The machine learning model provides a reward associated with the pre-trained reinforcement learning algorithm.

## Description

Various examples of the disclosure generally relate to determining surgical planning data for treating a disease, e.g., thermal ablation planning for treating tumors. Various examples of the disclosure specifically relate to fine-tuning a pre-trained reinforcement learning algorithm which is configured to determine surgical planning data for treating a disease associated with an anatomical target region.

Cancer is the second leading cause of death worldwide and can affect almost any tissue type and organ, e.g., liver, lung, kidney, bone, and so on. According to a report from the World Health Organization, in 2018, 1 in 6 or approximately 9.6 million deaths were attributed to cancer. Depending on the tumor (or cancer) progression and location, the therapeutic options may vary significantly. Examples include chemotherapy, radiotherapy, resection, or transplantation. One promising, minimally invasive therapy is thermal ablation, which aims at inducing tumor necrosis via thermal damage such as by inserting percutaneously one or more needles. For example, the thermal damage may be induced locally by extreme hyperthermia using electromagnetic energy, e.g., radiofrequency or microwave ablation, or by freezing the tissue by extreme hypothermia, i.e., cryoablation. Treatment success of thermal ablation is commonly achieved if the tumor as well as a safety margin, e.g., greater than 5 mm for liver tumors, is ablated [1, 2], which mitigates risks of local tumor recurrence. It is therefore important to precisely plan the intervention, i.e., determine surgical planning data.

Generally, surgical planning is a preoperative method of pre-visualising a surgical intervention in order to predefine surgical steps and/or configuration. Surgical planning facilitates an evaluation of complex anatomy and helps to enhance and speed up disease interpretation. Surgical planning, e.g., patient-specific planning, is also beneficial when results of the surgical planning are used in an operating room and offered to surgeons during interventions as a guide.

However, surgical planning such as planning thermal ablation procedures is challenging and time-consuming. For example, thermal ablation planning may comprise finding an appropriate count of electrodes, their skin entry and target points, as well as a suitable ablation configuration, e.g., power and duration in microwave ablation, while accounting for the complex three-dimension (3D) and irregularly shaped tumors, a large number of surrounding organs at risk, and additional organ-specific physiological and pathophysiological constraints that may alter the ablation zone, e.g., the cooling effect (heat sink) of major vessels in the liver. Thus, the parameter space of parameters subject to the planning has a high dimensionality.

The clinical practice of thermal ablation planning may comprise manual planning that considers for reference two-dimension (2D) views of pre-operative computed tomography (CT) images. For example, clinicians may perform manual planning based on CT images and the device manufacturer's recommendations on possible ablation zone sizes, which is extremely time-consuming.

An alternative is represented by computer-assisted planning algorithms which may facilitate the process of finding appropriate needle positions and configurations. Computer-assisted planning algorithms may provide faster and more robust results compared to the manual planning approach. A variety of algorithms for single and multiple-needle solutions have been proposed in recent years.

For example, non-patent literature [3] conducted a review on computer-assisted needle trajectory planning for radiofrequency ablation (RFA) and microwave ablation (MWA) of liver tumors. Fundamentals of needle trajectory planning are summarized. Algorithms for single-needle and multi-needle trajectory planning are analyzed.

Non-patent literature [4] discloses an automatic RFA planning method. First, a two-steps set cover-based model is formulated, which can integrate multiple clinical constraints for optimization of overlapping ablations. To ensure that the planning model can be solved in a reasonable time, a search space reducing strategy is then proposed. An algorithm for automatic RFA electrode selection, which provides a proper electrode ablation zone for the planning model, is also proposed.

Non-patent literature [5] discloses a tool called RF-Sim, being part of a complete 3D reconstruction and visualization project and including both a realistic radiofrequency ablation simulator for training and rehearsal, and an automatic treatment planner taking into account tumor's environment. They help radiologists to have a better visualization of patients' anatomic structures and pathologies and allow them to easily find an adequate treatment.

Non-patent literature [6] discloses an automated computation of optimal needle insertion in computer-assisted surgery with 3D visualization, which relies on a quasi-exhaustive search.

However, these methods disclosed in [4, 5, and 6] typically require testing a large number of hypotheses, and consequently, they are computationally expensive and hinder translation to the clinical environment. Possibilities for field deployment are limited. Moreover, the method disclosed in [4] returns a set of Pareto optimal solutions, i.e., two solutions, which makes it difficult to translate such a method into clinical settings. Because reviewing multiple needle plans would make the workflow significantly heavier and more complex instead of simplifying it. Ultimately, to return only a single solution, a cost function is defined that reflects the clinical constraints and prioritizes them.

One way to overcome the long running time of conventional planning algorithms is presented by deep reinforcement learning (DRL), in which an agent module, e.g., a deep neural network, learns to displace the needle(s) by interacting with a virtual patient environment. Such DLR planning algorithm significantly reduces the number of hypotheses to test. If the agent module is approaching an appropriate surgical planning, it receives a positive reward. On the other hand, if the clinical constraints are not satisfied, the agent module will receive negative rewards. For example, non-patent literature [7] proposes to leverage a DRL approach to find a suitable electrode trajectory that satisfies clinical constraints and does not require any labels in training.

However, such DRL approaches still suffer from some drawbacks. For example, there are variations among clinicians with respect to specific preferences, which makes it challenging to customize a potential approach for every clinician. As another example, different clinics or hospitals may have different distributions of patients, e.g., in terms of age, ethnicity, or gender, which may result in imbalanced data, and consequently, a specific DRL approach may be not able to precisely process such imbalanced data.

Accordingly, there is a need for advanced techniques which mitigate or overcome the above-identified drawbacks or restrictions. There is a need for advanced techniques of precise, reliable, and automatic determination of surgical planning data for treating a disease associated with an anatomical target region, e.g., liver cancer.

For example, such techniques may take into account clinicians' specific preferences and/or distributions of patients.

This need is met by the features of the independent claims. The features of the dependent claims define embodiments.

A computer-implemented method for fine-tuning a pre-trained reinforcement learning algorithm is provided. The pre-trained reinforcement learning algorithm is configured to determine surgical planning data for treating a disease associated with an anatomical target region. The method comprises obtaining one or more instances of the surgical planning data and obtaining one or more scores. Each of the one or more scores is associated with a quality of the respective instance of the surgical planning data. The method further comprises determining, based on each of the one or more instances of the surgical planning data and using a machine learning model, a respective estimated score associated with the quality of the respective instance of the surgical planning data. The method additionally comprises updating parameter values of the machine learning model based on a comparison between each of the one or more scores and a corresponding estimated score. The method also comprises fine-tuning the pre-trained reinforcement learning algorithm using the updated machine learning model for determining a reward associated with the pre-trained reinforcement learning algorithm.

A further computer-implemented method for determining surgical planning data for a treatment of a disease associated with an anatomical target region of a patient is provided. The method comprises obtaining one or more medical images. The one or more medical images depict the anatomical target region of the patient. The method further comprises determining, based on the one or more medical images, the surgical planning data using a reinforcement learning algorithm. Fine-tuning of the reinforcement learning algorithm is based on a reward determined based on a pre-trained machine-learning model.

For example, the reinforcement learning algorithm is fine-tuned by the method described above.

A computer program product or a computer program or a computer-readable storage medium including program code is provided. The program code can be executed by at least one processor. Executing the program code causes the at least one processor to perform either method described above.

A computing device comprising at least one processor and a memory is provided. Upon loading and executing program code from the memory, the at least one processor is configured to perform a method for fine-tuning a pre-trained reinforcement learning algorithm. The pre-trained reinforcement learning algorithm is configured to determine surgical planning data for treating a disease associated with an anatomical target region. The method comprises obtaining one or more instances of the surgical planning data and obtaining one or more scores. Each of the one or more scores is associated with a quality of the respective instance of the surgical planning data. The method further comprises determining, based on each of the one or more instances of the surgical planning data and using a machine learning model, a respective estimated score associated with the quality of the respective instance of the surgical planning data. The method additionally comprises updating parameter values of the machine learning model based on a comparison between each of the one or more scores and a corresponding estimated score. The method also comprises fine-tuning the pre-trained reinforcement learning algorithm using the updated machine learning model for determining a reward associated with the pre-trained reinforcement learning algorithm.

A further computing device comprising at least one processor and a memory is provided. Upon loading and executing program code from the memory, the at least one processor is configured to perform a method for determining surgical planning data for a treatment of a disease associated with an anatomical target region of a patient. The method comprises obtaining one or more medical images. The one or more medical images depict the anatomical target region of the patient. The method further comprises determining, based on the one or more medical images, the surgical planning data using a reinforcement learning algorithm. Fine-tuning of the reinforcement learning algorithm is based on a reward determined based on a pre-trained machine-learning model.

A medical imaging equipment is provided. The medical imaging equipment comprises a computing device comprising at least one processor and a memory. Upon loading and executing program code from the memory, the at least one processor is configured to perform either method described above.

It is to be understood that the features mentioned above and those yet to be explained below may be used not only in the respective combinations indicated, but also in other combinations or in isolation without departing from the scope of the disclosure.

### Brief description of the drawings

FIG. 1 schematically illustrates an exemplary image depicting an instance of surgical planning according to various examples.
FIG. 2 schematically illustrates a reinforcement learning algorithm according to various examples.
FIG. 3 schematically illustrates details with respect to a system according to various examples.
FIG. 4 schematically illustrates an exemplary network architecture of a machine learning model according to various examples.
FIG. 5 schematically illustrates a further exemplary network architecture of a machine learning model according to various examples.
FIG. 6 is a flowchart of a method according to various examples.
FIG. 7 is a block diagram of a device according to various examples.

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the disclosure will be described in detail with reference to the accompanying drawings. It is to be understood that the following description of embodiments is not to be taken in a limiting sense. The scope of the disclosure is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Various techniques disclosed herein generally relate to fine-tuning a pre-trained algorithm. For instance, a reinforcement learning algorithm may be fine-tuned. The algorithm obtained from such fine-tuning facilitates a determination of surgical planning for treating a disease associated with an anatomical target region.

According to various examples of the disclosure, the fine-tuning is specifically adapted: The pre-trained reinforcement learning algorithm is fine-tuned using an updated (or trained) machine learning model. The machine learning model provides, as an output, a reward associated with the pre-trained reinforcement learning algorithm. Parameter values of the machine learning model are updated as part of the fine-tuning - i.e., trained - based on a comparison between each of one or more scores associated with a quality of a respective instance of the surgical planning and a corresponding estimated score associated with the quality of the respective instance of the surgical planning, e.g., using supervised learning. Each of the estimated scores is determined using the machine learning model and based on a respective instance of surgical planning data.

The fine-tuned pre-trained reinforcement learning algorithm can be configured to process medical imaging data associated with an anatomical target region of a patient, e.g., the heart, the liver, the brain, and so on. In other examples, other kind of imaging data could be processed, e.g., projection imaging data, e.g., for security scanners or material inspection.

According to the disclosure, various kinds and types of medical imaging data may be processed. As a general rule, it would be possible that the fine-tuned pre-trained reinforcement learning algorithm and the pre-trained reinforcement learning algorithm can process 2D images or raw data obtained in K-space. The fine-tuned pre-trained reinforcement learning algorithm and the pre-trained reinforcement learning algorithm may process 3D depth data, e.g., point clouds or depth maps. Voxel data structures may be processed, e.g., as obtained from Computed Tomography or Magnetic Resonance Imaging (MRI). Either the fine-tuned pre-trained reinforcement learning algorithm or the pre-trained reinforcement learning algorithm may process time varying data, where one dimension stores an image or volume representation at different points in time.

Various techniques described herein generally relate to reinforcement learning. Reinforcement learning generally describes a machine-learning process associated with taking an appropriate action (here: e.g., how to determine surgical planning for treating a disease taking a clinical environment, e.g., anatomical and/or physiological conditions of the patient, into account) that maximizes a reward (here: e.g., outcomes of the surgery after a certain amount of time and/or a score associated with a quality of the surgical planning). Reinforcement learning is generally different from supervised learning: labeled training data, e.g., manually annotated training data, is not required; rather, reinforcement learning is enabled by monitoring outcomes of specific instances, i.e., by monitoring the reward. In the present case, disease treatment can be monitor, e.g., in the operating room.

Hereinafter, various examples will be described in the context of a (fine-tuned) pre-trained reinforcement learning algorithm configured for processing medical imaging data. However, similar techniques can be readily applied to other kinds and types of imaging data.

According to this disclosure, the pre-trained reinforcement learning algorithm may comprise any one of the available reinforcement learning algorithms before the filing of this application, e.g., the one disclosed in non-patent literature [7]. Other exemplary (pre-trained) reinforcement learning algorithms may comprise any one as disclosed in the following documents:
Non-patent literature - Ackermann J, Wieland M, Hoch A, Ganz R, Snedeker JG, Oswald MR, Pollefeys M, Zingg PO, Esfandiari H, Fürnstahl P. A new approach to orthopedic surgery planning using deep reinforcement learning and simulation. InMedical Image Computing and Computer Assisted Intervention-MICCAI 2021: 24th International Conference, Strasbourg, France, September 27-October 1, 2021, Proceedings, Part IV 24 2021 (pp. 540-549). Springer International Publishing. [8]
Non-patent literature - Rüttgers M, Waldmann M, Vogt K, Ilgner J, Schröder W, Lintermann A. Automated surgery planning for an obstructed nose by combining computational fluid dynamics with reinforcement learning. Computers in biology and medicine. 2024 May 1;173:108383. [9]
Non-patent literature - Ou Y, Tavakoli M. Sim-to-real surgical robot learning and autonomous planning for internal tissue points manipulation using reinforcement learning. IEEE Robotics and Automation Letters. 2023 Mar 10;8(5):2502-9. [10]
Non-patent literature - Zhang Q, Li M, Qi X, Hu Y, Sun Y, Yu G. 3D path planning for anterior spinal surgery based on CT images and reinforcement learning. In2018 IEEE international conference on cyborg and bionic systems (CBS) 2018 Oct 25 (pp. 317-321). IEEE. [11]

Hereinafter, the techniques of this disclosure will be described in connection with an exemplary reinforcement learning algorithm for automatic planning of liver tumor thermal ablation such as those disclosed in US patent application 2024/0115320 A1, which is incorporated herein by reference. I.e., the surgical planning may comprise a thermal ablation planning which may comprise a determination of at least one of the following: an insertion point for an ablation needle, a trajectory for inserting the ablation needle, a safety margin, a target point, an ablation zone, a contour of skin, a contour of a tumor, and one or more ablation configurations.

In general, thermal ablation may include hyperthermic ablation (e.g., radiofrequency ablation, microwave ablation, laser ablation, and high-intensity focused ultrasound ablation) and hypothermic ablation (cryoablation). Radiofrequency ablation (RFA) and microwave ablation (MWA) have become the main ablation treatments for liver tumors.

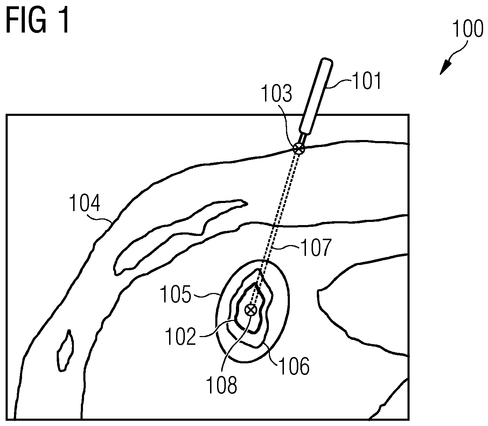

FIG. 1 schematically illustrates an exemplary image 100 depicting an instance of surgical planning data, e.g., for RFA or MWA, RFA and MWA. These are minimally invasive therapies which may involve an ablation applicator or needle 101 (i.e., radiofrequency electrode or microwave antenna) inserted percutaneously into a tumor 102, e.g., a liver tumor, via an insertion point 103 on the surface of the skin 104 to destroy the tumor 102 in situ by heating-induced coagulation necrosis. A heating-induced ablation zone 105 may be generated to cover the tumor 102 with a 5-10 mm safety margin 106. Optionally or alternatively, a needle trajectory 107, i.e., a trajectory for inserting the ablation needle 101, may be generated. The needle trajectory 107 may be a line segment bounded by the insertion point 103 and a target point 108 within the tumor 102. The target point 108 may represent a position of the tip of the respective ablation electrode 101. For example, the target point 108 may be the center or the centroid of the tumor 102.

According to various examples, medical imaging, such as CT and/or MRI, may be used to determine surgical planning data for RFA and MWA procedures and provide guidance during such procedures.

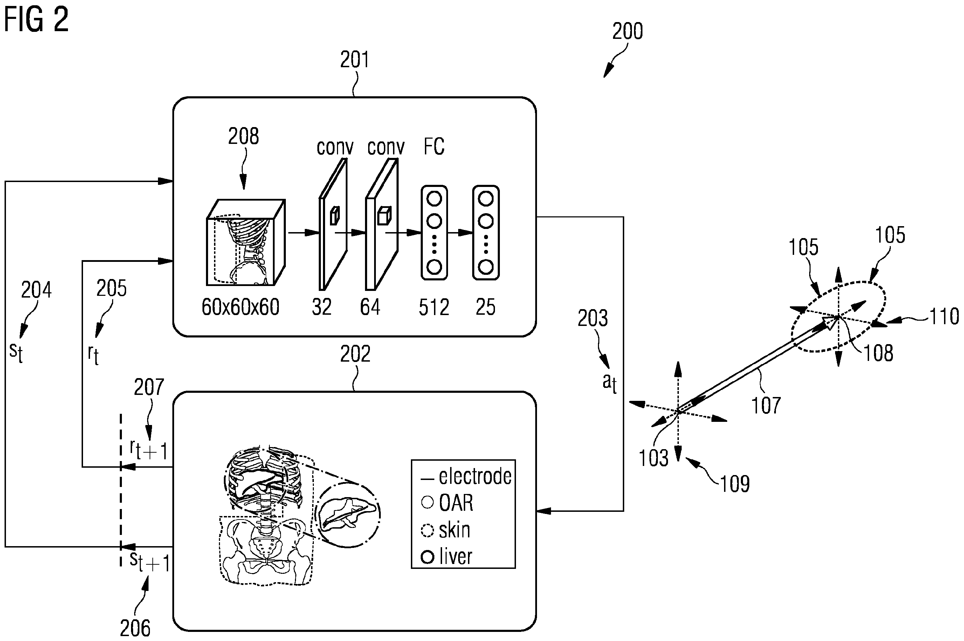

According to various examples, a trained or pre-trained or fined-tuned reinforcement learning algorithm, e.g., the reinforcement learning algorithm 200 shown in FIG. 2, may be used to automatically determine surgical planning data for RFA and MWA, e.g., finding an appropriate insertion point 103 and/or an appropriate target point 108, i.e., an appropriate needle trajectory 107 for the ablation applicator or needle 101.

While in the scenario of FIG. 1, the surgical planning data is implemented by a 2D image, as a general rule, other data formats may be used for implementing the surgical planning data. For instance, points in 3D space or curves in 3D space may be used to define certain structures, positions or trajectories. Then, a subsequent 3D or 2D rendering may be required to generate an image as shown in FIG. 1.

In general, surgery comprises conventional open surgery, minimally-invasive surgery, and hybrid surgery using a combination of open and minimally-invasive techniques in terms of degree of invasiveness. A surgery can be performed by one or more surgeons or one or more interventional radiologists. A surgery can also be performed by one or more surgeons together with one or more interventional radiologists. Surgical planning may comprise conventional open surgery planning, minimally-invasive surgery planning, e.g., planning for intervention such as ablation, and hybrid surgery planning.

FIG. 2 schematically illustrates aspects with respect to a reinforcement learning algorithm 200. The reinforcement learning algorithm 200 may be configured to determine surgical planning data for treating a liver tumor using thermal ablation. For example, the reinforcement learning algorithm 200 may be configured to determine trajectories of one or more ablation applicator or needle 101 for performing a thermal ablation on one or more tumors 102.

The reinforcement learning algorithm 200 may comprise an agent module 201. The agent module 201 may comprise one or more agents and each of the one or more agents may iteratively update the trajectories of the one or more ablation applicators 101 within a clinical environment 202 by determining one or more actions (*aₜ*) 203 based on a current state (*sₜ*) 204 and further based on a current reward (*rₜ*) 205 defined based on clinical constraints. Based on the updated trajectories of the respective ablation electrode 101 in the environment 202, the current state 204 is updated to an updated or new state (*s*_{*t*+1}) 206, and the current reward 205 is updated to an updated or new reward (*r*_{*t*+1}) 207. The objective is to iteratively maximize the reward 205 by learning an optimal or appropriate policy that gives a set of actions 203 for updating the current trajectories of the one or more ablation applicators 101 within the clinical environment 202 to reach an appropriate state from the current state.

According to various examples, the one or more actions 203 may comprise displacing the insertion point 103 and/or the target point 108 by, e.g., a distance ranging from 1 voxel to 5 voxels in any direction of Cartesian coordinate systems 109 and 110, respectively. Optionally or additionally, the one or more actions 203 may comprise determining displacements of the ablation zone 105, which is shown as an ellipsoid in FIG. 2.

According to various examples, the reinforcement learning algorithm 200 may be trained to satisfy a set of clinical constraints comprising hard and soft constraints.

For example, the hard constraints may comprise at least one of the following:
- a length of a respective needle trajectory 107 being smaller or equal to the maximum needle length;
- no organ at risk (OAR) being injured;
- a resulting ablation completely covering the tumor 102 and the safety margin 106; and
- the respective needle trajectory going through at least 1cm of healthy liver tissue.

The soft constraints may comprise at least one of the following:
- the needle trajectory being in-plane to a CT scan;
- the needle trajectory being on top of the rib;
- the needle trajectory having a minimal length; and
- the needle trajectory maximizing the distance to risk structures, e.g., OAR.

According to various examples, the clinical environment 202 may illustrate or define the current state (*sₜ*) 204. For example, the clinical environment 202 may comprise 3D information of medical imaging data associated with an anatomical target region of a patient, e.g., the liver, information of a segmented image depicting one or more tissues within the anatomical target region, e.g., contours of the liver, the ribs, the skin, and/or OAR, and/or one or more current trajectories of the respective ablation electrode 101. Optionally or additionally, the clinical environment 202 may comprise respective contours of one or more ablation zones 105, e.g., shown as one or more ellipsoids (not shown in FIG. 2). As shown in FIG. 2, the current state (*sₜ*) 204 may be determined based on the segmented image depicting the one or more tissues within the anatomical target region, e.g., tumors 102, OAR, and skin 104, and the one or more current trajectories 107 of the respective ablation electrode 101. Optionally or additionally, the current state (*sₜ*) 204 may be determined further based on respective contours of one or more ablation zones 105.

According to various examples, each of the one or more agents (only one shown in FIG. 2) of the agent module 201 may comprise a deep Q-network (DQN) as presented in non-patent literature - Mnih V, Kavukcuoglu K, Silver D, Rusu AA, Veness J, Bellemare MG, Graves A, Riedmiller M, Fidjeland AK, Ostrovski G, Petersen S. Human-level control through deep reinforcement learning. nature. 2015 Feb 26;518(7540):529-33. [12]. For example, each of the DQN may take one or more medical images (e.g., 60*60*60 voxels) as input 208, followed by several convolutional layers conv (two convolutional layers shown in FIG. 2) and one fully connected layers FC with output of respective actions.

According to various examples, each of the one or more agents, e.g., the DQN, learns in a gamified world by repeatedly displacing electrodes 101 in a virtual clinical environment 202. The agent may observe the current state 204 of the environment 202, e.g., a volume denoting one or more risk structures, the skin surface 104, the treatment location (e.g., the tumor 102 and the safety margin 106), the (expected) ablation zone 105, and the needle trajectory 107. Based on the current state 204, the DQN may propose which action to take, i.e., how to change the position of the insertion point 103 and/or the target point 108. The action may update the environment 202 and provide again the new state 206 and a new reward 207 to the agent. The reward may be descriptive of the task at hand, i.e., it may be positive and large if the agent approaches the optimal/appropriate configuration and may be negative and very low if the clinical constraints are not satisfied.

Various techniques are based on the finding that reference techniques - in which the reward is calculated using a pre-defined function - face certain restrictions and drawbacks. For instance, it is extremely challenging to define or formulate an appropriate reward function that is informative to the agent module 201 and that reflects the clinical constraints and optimality of surgical planning data, e.g., the thermal ablation planning. Moreover, different clinicians may have specific preferences, which makes it even more challenging to customize a reinforcement learning algorithm for every clinician.

These drawbacks are mitigated by the techniques described herein by fine-tuning a pre-trained reinforcement learning algorithm (reinforcement learning algorithm may be equally referred to as reinforcement learning model using a machine learning model for determining a reward associated with the pre-trained reinforcement learning algorithm. In other words, the machine learning model implements the reward function required for fine-tuning of the reinforcement learning algorithm. Optionally or additionally, the pre-trained reinforcement learning algorithm may be further fine-tuned based on training data collected in specific sites, e.g., hospitals or institutions, and/or by specific clinicians. It is also possible to fine-tune the pre-trained reinforcement learning algorithm upon receiving or obtaining new training data.

After fine-tuning the pre-trained reinforcement learning algorithm, the fine-tuned reinforcement learning algorithm can be deployed, e.g., in various hospitals or institutions, to determine surgical planning data for patients. It is also possible to continuously fine-tune instances of the fined-tuned pre-trained reinforcement learning algorithm at different deployment locations based on new training data.

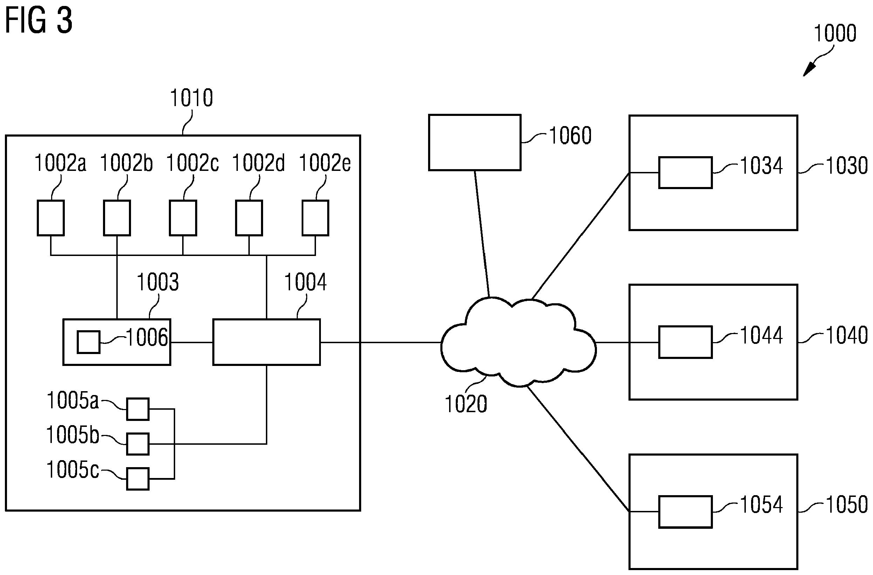

FIG. 3 schematically illustrates details with respect to a system 1000 according to various examples. The system 1000 may include four local networks 1010, 1030, 1040, as well as 1050, which are respectively within four hospitals or institutions, and a(n) external/shared network 1020, such as the internet or a cloud, via which the four local networks 1010, 1030, 1040, as well as 1050 may be communicated with each other. FIG. 3 is only an illustration of one possible example; generally, the number of local networks may be any positive integer, e.g., 1, 2, 3 and so on. The external/shared network 1020 is optional.

Each of the four local networks 1010, 1030, 1040, as well as 1050 may share the same or similar architecture and have the same or similar network elements or devices. For example, the local network 1010 may comprise at least one medical imaging equipment 1002a-1002e, at least one local data repository 1003 comprising a picture archiving and communication system (PACS) 1006, at least one computing device 1004 connectable to the external/shared network 1020. The computing device 1004 can act as a gateway node to connect to the outside of the local network 1010, e.g., to any network node connectable via the external/shared network 1020. For example, the computing device 1004 can connect, via the external/shared network 1020, to respective computing devices 1034, 1044, and 1054 of respective local networks 1030, 1040, and 1050. Similarly, the respective computing devices 1034, 1044, and 1054 can also act as respective gateway nodes of the respective local networks 1030, 1040, and 1050. The local network 1010 further comprises at least one user terminal 1005a-1005c, which is generally optional. Within the local network 1010, each of the at least one medical equipment 1002a-1002e is respectively connectable to the at least one local data repository 1003 and the at least one computing device 1004 via physical cables or via wireless communication; each of the at least one user terminal 1005a-1005c may be connectable to the at least one computing device 1004 via physical cables or via wireless communication.

According to various examples, the medical imaging equipment 1002a-1002e comprises one or more of an X-ray scanner, a computed tomography scanner, a magnetic resonance imaging scanner, a positron emission tomography scanner, an ultrasound scanner, and so on. A medical imaging examination of a patient can be performed by a radiologist using at least one of the medical imaging equipment 1002a-1002e, with respect to at least one anatomical target region of the patient. Medical imaging data are obtained by the medical imaging examination and may be encoded according to a standard, such as the Digital Imaging and Communications in Medicine (DICOM) standard. Other standards, such as Joint Photographic Experts Group (JPEG) or Tagged Image File Format (TIFF), may be used. The (encoded) medical imaging data may be transmitted to the at least one local data repository 1003 and/or the at least one computing device 1004. The (encoded) medical imaging data may be stored in the at least one local data repository 1003 and/or in the at least one computing device 1004.

According to this disclosure, one or more instances of surgical planning data for treating a disease associated with an anatomical target region may be determined by a radiologist (and/or other medical practitioners) during a medical imaging examination of a patient, such as an ultrasound examination or an angiography examination. Additionally or alternatively, the one or more instances of surgical planning data may be determined by the radiologist (and/or other medical practitioners) after the medical imaging examination by reviewing/studying the medical imaging data acquired during the medical imaging examination. For example, the radiologist may obtain, via one of the at least one user terminal 1005a-1005c, the medical imaging data from the at least one local data repository 1003 or the at least one computing device 1004, and determine the one or more instances of surgical planning data. It is also possible to use a pre-trained reinforcement learning algorithm described herein to determine the one or more instances of surgical planning data by feeding the medical imaging data to an agent module of the pre-trained reinforcement learning algorithm, e.g., 200 of FIG. 2. After determining the one or more instances of surgical planning data, the one or more instances of surgical planning data may be also stored in the at least one local data repository 1003 and/or in the at least one computing device 1004. Each of the one or more instances of surgical planning data may be represented or illustrated by a respective image, e.g., image 100 shown in FIG. 1.

According to various examples, for each of the one or more instances of surgical planning data, a respective score associated with a quality of the respective instance of the surgical planning data may be determined, e.g., by one or more clinicians. It is also possible to use a reward function to determine an estimation of the respective score and the respective score may be determined by one or more clinicians based on revision or adjustment of the estimation of the respective score.

According to various examples, the (fine-tuned) pre-trained reinforcement learning algorithm outlined above may be executed by the at least one computing device 1004 or by a respective computing device embedded in or connected to a respective medical equipment 1002a-1002e. When new medical imaging data are acquired, a respective device managing the (fine-tuned) pre-trained reinforcement learning algorithm may receive a trigger or notification, e.g., from the computing device 1004 or the local data repository 1003. Alternatively or additionally, the respective device managing the (fine-tuned) pre-trained reinforcement learning algorithm may proactively check, from time to time, whether new medical imaging data are available. Then, the (fine-tuned) pre-trained reinforcement learning algorithm may obtain medical imaging data and process the medical imaging data to determine surgical planning data for treating a disease.

It is also possible to continuously fine-tune the pre-trained reinforcement learning algorithm using new medical imaging data and ground-truth surgical planning data based on techniques disclosed herein.

According to this disclosure, if an instance of the pre-trained reinforcement learning algorithm is respectively executed by a respective node in the four local networks 1010, 1030, 1040, and 4050, such as computing devices 1004, 1034, 1044, and 1054, federated learning or distributed learning may be utilized to further fine-tune the pre-trained reinforcement learning algorithm.

Optionally, the system 1000 may comprise a central computing device 1060 which may be accessible to at least the respective computing devices 1004, 1034, 1044, and 1054 of the respective local networks 1010, 1030, 1040, and 1050. In some examples, each pair of the computing devices 1004, 1034, 1044, and 1054 may not be connectable directly, but can exchange data/information via the central computing device 1060, and thereby security of data generated/stored in the respective local networks 1010, 1030, 1040, and 1050 can be improved, for example by implementing access control techniques at the central computing device 1060. Further, the central computing device 1060 may facilitate a centralized federated learning or fine-tuning of the (pre-trained) reinforcement learning algorithm respectively executed by a respective node in the four local networks 1010, 1030, 1040, and 4050.

For example, the reinforcement learning algorithm 200 of FIG. 2 may be trained or pre-trained via the central computing device 1060 using training data available locally. Then, an instance of the trained or pre-trained reinforcement learning algorithm 200, e.g., the trained or pre-trained agent module 201, may be rolled out to a respective node in the four local networks 1010, 1030, 1040, and 4050 and used in clinical practice. For example, the instance of the trained or pre-trained reinforcement learning algorithm 200 may be executed by the computing devices 1034, 1044, and 1054, respectively. Optionally or additionally, the trained or pre-trained reinforcement learning algorithm 200 may be respectively fine-tuned by the computing devices 1034, 1044, and 1054 locally using data available at the respective computing device. Thus, the trained or pre-trained reinforcement learning algorithm 200 can be tailored or fine-tuned to specific data and/or requirements of specific hospitals or clinicians.

According to the disclosure, a method for fine-tuning a pre-trained reinforcement learning algorithm, e.g., the algorithm 200 of FIG. 2, is provided. The pre-trained reinforcement learning algorithm is configured to determine a surgical planning data for treating a disease associated with an anatomical target region. The method may be independently executed by any one of the computing devices 1004, 1034, 1044, and 1054. The pre-trained reinforcement learning algorithm is fine-tuned using an updated (or trained) machine learning model configured for determining a reward associated with the pre-trained reinforcement learning algorithm. I.e., the reward is determined using the updated (or trained) machine learning model when fine-tuning the reinforcement learning algorithm. On the other hand, the reward is determined using a pre-defined function when performing the pre-training of the reinforcement learning algorithm.

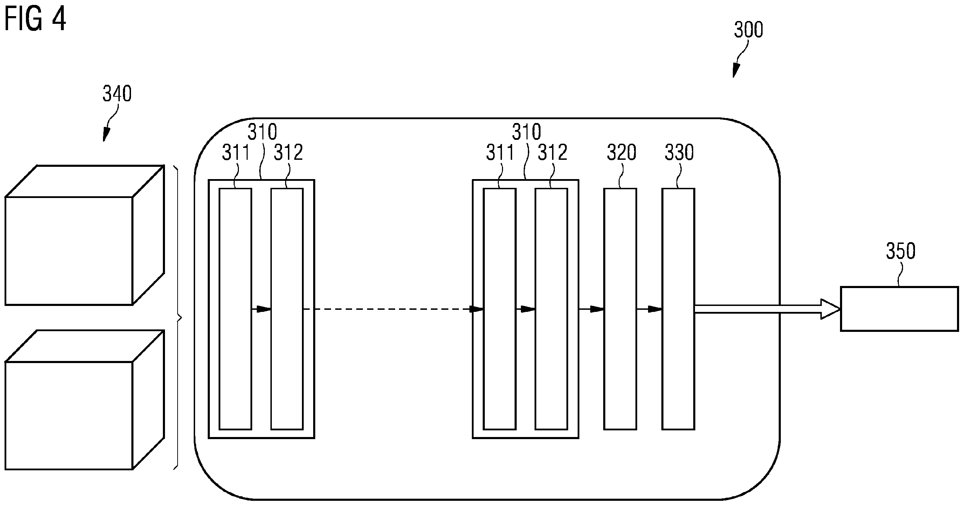

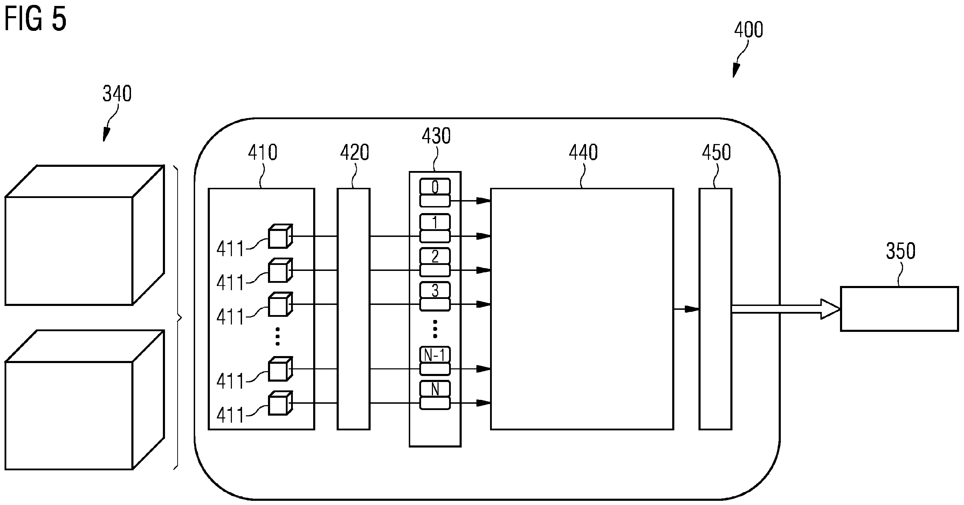

According to various examples, the machine learning model for determining a reward associated with the pre-trained reinforcement learning algorithm may comprise a convolutional neural network, e.g., as shown in FIG. 4, or a transformer-based neural network, e.g., as shown in FIG. 5.

FIG. 4 schematically illustrates an exemplary network architecture of the machine learning model for determining a reward associated with the pre-trained reinforcement learning algorithm, e.g., 200 of FIG. 2. The network architecture is based on a convolutional network 300. The convolutional network 300 may comprise one or more convolutional blocks 310 and each of the one or more convolutional blocks 310 may comprise one or more convolutional layers 311 followed by one or more pooling layers 312. The one or more convolutional blocks 310 may be followed by a flatten layer 320 and a fully-connected block 330 comprising one or more fully-connected layers.

According to various examples, the machine learning model for determining a reward associated with the pre-trained reinforcement learning algorithm, e.g., the convolutional network 300, may be trained using supervised learning algorithms. For example, the convolutional network 300 may be trained using a respective node in the four local networks 1010, 1030, 1040, and 4050, such as computing devices 1004, 1034, 1044, and 1054. For example, one or more images (2D or 3D) as input 340, in which each of the one or more images embodies a respective instance of the surgical planning data, may be fed to the convolutional network 300. Based on each of the one or more images and using the convolutional network 300, a respective estimated score associated with the quality of the respective instance of the surgical planning data 350, e.g., an estimated reward, is determined. Parameter values of the convolutional network 300 are updated/trained based on a comparison between each of one or more ground-truth scores and a corresponding estimated score. For example, the ground-truth scores may be determined by one or more clinicians manually.

FIG. 5 schematically illustrates a further exemplary network architecture of the machine learning model for determining a reward associated with the pre-trained reinforcement learning algorithm, e.g., 200 of FIG. 2. The network architecture is based on transformer-based network 400. The transformer-based network 400 may comprise a tokenizer module 410 including multiple tokenizers 411. The tokenizer module may split the input 340, e.g., one or more images (2D or 3D), into multiple (fixed-size) patches. The transformer-based network 400 may further comprise one or more linear projection layers 420, e.g., one or more fully-connected layers, following the tokenizer module 410. The one or more linear projection layers 420 linearly embed each of the multiple fixed-size patches into a respective patch embedding. Then, a sequence of embedding vectors 430 is obtained by adding a position embedding to a respective patch embedding. The respective position embedding retains position information of a corresponding patch. The sequence of embedding vectors 430 is fed to a transformer encoder 440 followed by a multiple-layer perceptron 450, e.g., including multiple fully-connected layers. The multiple-layer perceptron 450 produces output, e.g., a respective estimated score associated with the quality of the respective instance of the surgical planning data 350, e.g., an estimated reward.

Alternatively, the machine learning model for determining a reward associated with the pre-trained reinforcement learning algorithm may comprise a further transformer-based network architecture as presented in non-patent literature - Dosovitskiy A, Beyer L, Kolesnikov A, Weissenborn D, Zhai X, Unterthiner T, Dehghani M, Minderer M, Heigold G, Gelly S, Uszkoreit J. An image is worth 16x16 words: Transformers for image recognition at scale. arXiv preprint arXiv:2010.11929. 2020 Oct 22. [13]

In general, the transformer-based network 400 may be trained using the same techniques for training the convolutional network 300 as described above, e.g., using supervised learning algorithms.

After performing the training of the machine learning model, e.g., either the convolutional network 300 or the transformer-based network 400, the trained machine learning model can be used to determine a reward associated with the pre-trained reinforcement learning algorithm when performing the fine-tuning of the pre-trained reinforcement learning algorithm.

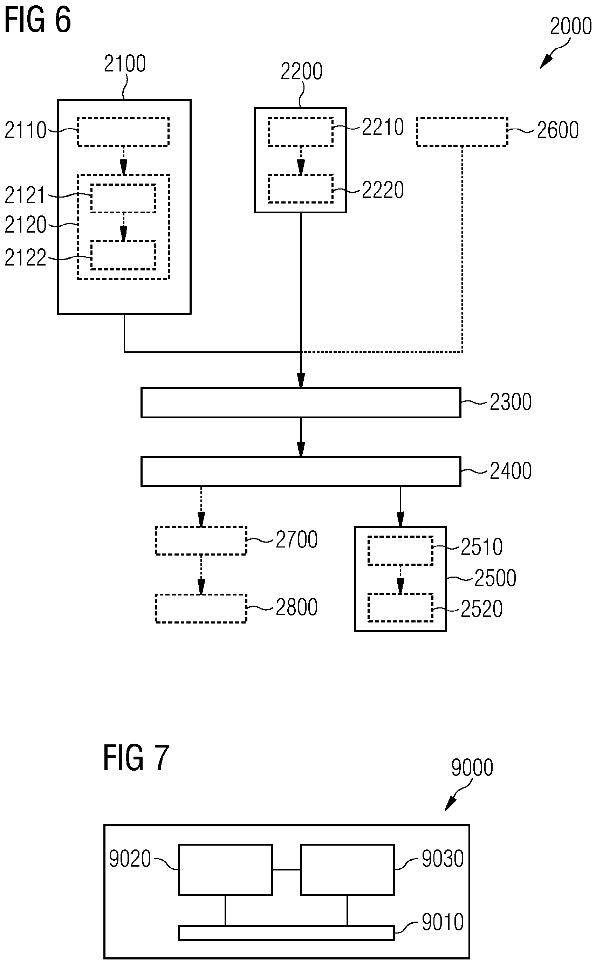

FIG. 6 is a flowchart of a method 2000 according to various examples. The method 2000 pertains to fine-tuning a pre-trained reinforcement learning algorithm. The pre-trained reinforcement learning algorithm is configured to determine surgical planning data for treating a disease associated with an anatomical target region. The pre-trained reinforcement learning algorithm is fine-tuned using an updated (or trained) machine learning model configured for determining a reward associated with the pre-trained reinforcement learning algorithm. Parameter values of the machine learning model are updated/trained based on a comparison between each of one or more scores associated with a quality of a respective instance of the surgical planning data and a corresponding estimated score associated with the quality of the respective instance of the surgical planning data, e.g., using supervised learning. Thus, the machine learning model may be repetitively retrained in order to provide a reward function for the fine-tuning of the pre-trained reinforcement learning algorithm that more accurately reflects the domain specific properties of certain users or hospitals.

Each of the estimated scores is determined using the machine learning model and based a respective instance of the surgical planning data. As a general rule, the surgical planning data may be embodied by an image that carries respective information. Alternatively or additionally, the surgical planning data may also be available in other data formats, e.g., trajectories or paths defined by coordinates in 3D space; then, a subsequent visualization protocol / rendering protocol may be required to generate, e.g., 2D images or 3D images.

The method 2000 can be executed by at least one processor upon loading program code. For example, the method 2000 could be executed by a processor of any one of the computing devices 1004, 1034, 1044, and 1054, upon loading program code from a respective memory. Details with respect to such a method will be explained below.

Optional boxes are labeled with dashed lines.

Block 2100: obtaining one or more instances of the surgical planning data. For instance, multiple 2D images may be obtained that each embody respective surgical planning data.

Block 2200: obtaining one or more scores, each of the one or more scores being associated with a quality of the respective instance of the surgical planning data.

According to various examples, block 2100 and block 2200 may be performed together. For example, multiple 2D images, e.g., image 100 shown in FIG. 1, or 3D images, which respectively implement multiple instances of the surgical planning data, may be obtained by manually annotated by one or more clinicians based on medical imaging data acquired using at least one of the medical imaging equipment 1002a-1002e. Such 2D or 3D images may be stored in the at least one local data repository 1003. Scores associated with a quality of the multiple instances of the surgical planning data may be also provided by the one or more clinicians and stored in the at least one local data repository 1003. The multiple instances of the surgical planning data may be respectively linked to the scores.

For example, the one or more images and the one or more scores may be obtained by exploiting local data, e.g., within any one of the local networks 1010, 1030, 1040, as well as 1050 of FIG. 3. For example, the local data may comprise patient data of already performed thermal ablations, e.g., medical images, needle positions/trajectories, and ablation protocol.

Alternatively or optionally, it is also possible to separately obtain the one or more images and the one or more scores. I.e., block 2100 and block 2200 may be performed separately or in parallel.

Optionally or additionally, i.e., block 2100, may comprise: block 2110, obtaining one or more medical images, each of the one or more medical images depicting the anatomical target region, e.g., the liver; and block 2120, determining, based on the one or more medical images, the one or more images using the pre-trained reinforcement learning algorithm.

For example, the medical images may be obtained or reconstructed based on medical imaging data. The medical imaging data could be acquired using multiple configurations of medical imaging equipment 1002a-1002e, or using multiple imaging equipment. Various imaging modalities are conceivable. For instance, different parameters for the acquisition of the medical imaging data, e.g., exposure time, MRI scanning protocol, CT contrast, etc. could be selected. The obtained one or more medical images may be 2D images or 3D images, e.g., 208 of FIG. 2. The obtained one or more medical images may be processed using the pre-trained reinforcement learning algorithm, e.g., 200 of FIG. 2, to determine the one or more instances of the surgical planning data, e.g., image 100 of FIG. 1. For example, the obtained one or more instances of the surgical planning data may be fed into the agent module 201 of the pre-trained reinforcement learning algorithm 200 and one or more sets of actions 203 are output from the agent module 201. The one or more instances of the surgical planning data such as 100 of FIG. 1 may be obtained based on the one or more sets of actions 203 and the clinical environment 202. Optionally or additionally, the obtained one or more instances of the surgical planning data may be manually adapted manually by clinicians based on specific preferences.

According to various examples, said determining of the one or more instances of the surgical planning data using the pre-trained reinforcement learning algorithm, i.e., block 2120, may comprise: block 2121, determining, based on the one or more medical images, multiple candidate instances of the surgical planning data using the pre-trained reinforcement learning algorithm; and block 2122, selecting, from the multiple candidate instances of the surgical planning data, the one or more instances of the surgical planning data based on a pre-defined criterion.

For example, the agent module 201 of the pre-trained reinforcement learning algorithm 200 may propose multiple sets of candidate actions and the multiple candidate images may be obtained based on the multiple sets of candidate actions and the clinical environment 202. For example, the agent module 201 may propose multiple instances of suitable surgical planning data, e.g., needle electrode placement(s) or trajectories and ablation setting (e.g., ablation duration), which are shown to the clinician. The clinician may rank from best to worst the proposed multiple instances of suitable surgical planning data. Alternatively, the clinician could classify the multiple instances of suitable surgical planning data as being either good or bad. The one or more instances of the surgical planning data may be selected based on the ranking or from the surgical planning data being classified as good.

According to various examples, said obtaining of the one or more scores, i.e., block 2200, may comprise: block 2210, obtaining a reference surgical planning data as ground-truth of the surgical planning data; and block 2220, determining the one or more scores based on a comparison between the reference surgical planning data and a corresponding instance of the surgical planning data.

For example, the one or more scores may be determined based on a structural similarity measure between the reference surgical planning data and an instance of the surgical planning data, e.g., image 100 of FIG. 1. This may be an image-to-image comparison. Such a structural similarity measure may comprise those disclosed in a non-patent literature - Wang Z, Bovik AC, Sheikh HR, Simoncelli EP. Image quality assessment: from error visibility to structural similarity. IEEE transactions on image processing. 2004 Apr 13;13(4):600-12. [14]. It is also possible to determine the one or more score based on a distance between a needle trajectory depicted in the reference surgical planning data and a further needle trajectory depicted in the corresponding instance of the surgical planning data. For instance, distances in 3D space may be determined. For instance, shape differences may be considered. Such comparisons may not necessitate an image-to-image comparison. Additionally or optionally, the one or more score can be determined further based on outcome information, e.g., whether tumor recurrence happened after a certain amount of time.

Block 2300: determining, based on each of the one or more instances of the surgical planning data and using a machine learning model, a respective estimated score associated with the quality of the respective instance of the surgical planning data.

For example, the machine learning model may be either the convolutional network 300 of FIG. 4 or the transformer-based network 400 of FIG. 5.

Optionally or additionally, the method 2000 may further comprise block 2600, obtaining a segmented image depicting one or more tissues within the anatomical target region. Said determining of the respective estimated score associated with the quality of the respective instance of the surgical planning data, i.e., block 2300, is further based on the segmented image.

For example, the segmented image may depict contours of the one or more tissues within the anatomical target region, e.g., the liver, the ribs, the skin, and/or OAR, and/or one or more current trajectories of the respective ablation electrode 101.

Block 2400: updating parameter values of the machine learning model based on a comparison between each of the one or more scores and a corresponding estimated score. Thus, a retraining of the machine learning model is implemented. Such retraining may be repeatedly implemented, responsive to obtaining additional instances of the surgical planning data and associated scores.

As a general rule, at least one loss function, e.g., L1 loss, L2 loss, or Laplacian pyramid loss, may be used to update the parameter values of the machine learning model. Backpropagation may be used to implement a gradient-descent optimization of the weights of the machine learning model.

Block 2500: fine-tuning the pre-trained reinforcement learning algorithm using the updated machine learning model for determining a reward associated with the pre-trained reinforcement learning algorithm.

According to various examples, said fine-tuning of the pre-trained reinforcement learning algorithm, i.e., block 2500, may comprise: block 2510, obtaining one or more further medical images, each of the one or more further medical images depicting the anatomical target region; and block 2520, processing the one or more further medical images using an agent module and an environment module of the pre-trained reinforcement learning algorithm together with the updated machine learning model.

In general, the fine-tuning of the pre-trained reinforcement learning algorithm is performed in the same way as the initial training a reinforcement learning algorithm, with the difference that the reward is determined using the trained machine learning algorithm, e.g., network 300 or 400, rather than using a pre-defined reward function. During the fine-tuning, weights of the pre-trained reinforcement learning algorithm are adjusted starting from there values obtained from the initial training.

According to various examples, by training the machine learning model 300 or 400 locally at each of the four hospitals where the four local networks 1010, 1030, 1040, as well as 1050 are located, the trained machine learning model 300 or 400 can be tailored to hospital-specific or clinician-specific preferences. The trained machine learning model may then be used to fine-tune the pre-trained reinforcement learning algorithm 200 locally at a specific hospital based on locally available patient datasets.

According to various examples, the fine-tuning of the pre-trained reinforcement learning algorithm, e.g., using retraining techniques, may be done in small incremental developments in a hospital or institution which are usually based on limited feedback from the hospital or institution. The fine-tuning of the pre-trained reinforcement learning algorithm is a sensitive matter which might improve the algorithm performance towards certain datasets, e.g., medical imaging data acquired in a specific hospital or institution, but it might not be representative of the overall data available in the field, i.e., the reinforcement learning algorithm may be overfitted. To solve the overfitting issues, medical imaging data acquired in different hospitals/institutions may be shared, however, medical imaging data are usually very big, and consequently, medical imaging data sharing is time-consuming. Further, country-specific regulations may prohibit sharing medical imaging data due to privacy.

Optionally, after performing a local fine-tuning of the pre-trained reinforcement learning algorithm within a hospital or institution, if the same pre-trained reinforcement learning algorithm is also utilized in other hospitals or institutions, for example, executed by at least two of the computing devices 1004, 1034, 1044, and 1054, federated learning may be applied to retrain or further fine-tune the pre-trained reinforcement learning algorithm to mitigate overfitting, to reduce overhead associated with medical imaging data sharing, and to protect privacy. Further, federated learning also can improve performances of the pre-trained reinforcement learning algorithm running in a small clinic or hospital which provides medical services to a small number of patients by sharing parameters of the pre-trained reinforcement learning algorithm revised/tailored in big hospitals or university medical centers.

Optionally or additionally, the method 2000 may further comprise: block 2700, providing, to a central computing device, e.g., the central computing device 1060 of FIG. 3, the updated parameters of the updated machine learning model; and block 2800, upon providing the updated parameters, receiving, from the central computing device 1060, an update of the updated machine learning model 300 or 400 and/or an update of the pre-trained reinforcement learning algorithm 200.

According to various examples, the update of the updated machine learning model may be performed, by the central computing device 1060, using secure aggregation and/or federated averaging based on the updated parameters of the updated machine learning model and further based on at least one additional update of the parameters of the machine-learning model, the at least one additional update of the parameters being received by the central computing device from one or more additional computing devices running the pre-trained reinforcement learning algorithm.

According to various examples, the update of the pre-trained reinforcement learning algorithm may be performed, by the central computing device, based on the update of the updated machine learning model for determining the reward associated with the pre-trained reinforcement learning algorithm.

To exploit shared insights among all hospitals, which could enable the next generation of improved agent module 201, each hospital-specific update of the machine learning model 300 or 400 and possibly also the reinforcement learning agent 200 could be aggregated globally by means of any federated learning mechanism. For example, this could entail aggregating the gradient information on the centralized server 1060 and updating the machine learning model 300 or 400 globally with standard gradient descent updates. The benefit of this specific instance of deep federated learning is that no patient information needs to be exchanged with the centralized remote server. It thus bodes well for the setup in hospitals with strict data policies such as hospitals underlying the General Data Protection Regulation. In addition, the training of the machine learning model 300 or 400 may take into account insights from clinicians, which could further improve or facilitate the performance of the reinforcement learning model. Further, the agent module 201, in principle, could be trained on the entirety of the hospital data lake, because no ground truth information is required.

Once the pre-trained reinforcement learning algorithm 200 is fined-tuned by the method 2000 described above, the fine-tuned reinforcement learning algorithm 200 can be used to determine, based on one or more medical images, a surgical planning data for a treatment of a disease associated with an anatomical target region of a patient. For instance, multiple 2D images including information for surgical planning may be generated by the fine-tuned reinforcement learning algorithm 200.The one or more medical images depict the anatomical target region of the patient.

For example, the one or more medical images may be obtained or reconstructed based on medical imaging data. The medical imaging data could be acquired using multiple configurations of medical imaging equipment 1002a-1002e, or using multiple imaging equipment. For instance, different parameters for the acquisition of the medical imaging data, e.g., exposure time, MRI scanning protocol, CT contrast, etc. could be selected.

FIG. 7 is a block diagram of a device 9000 according to various examples. The device 9000 may comprise at least one processor 9020, at least one memory 9030, and at least one input/output interface 9010. The at least one processor 9020 is configured to load program code from the at least one memory 9030 and execute the program code. Upon executing the program code, the at least one processor 9020 performs the method 2000.

Additionally or alternatively, the device 9000 may be embedded in any one of the medical imaging equipment 1002a-1002e of FIG. 3, and thereby the medical imaging equipment may be also configured to perform the method 2000.

Summarizing, techniques have been described that facilitate precise, reliable, and automatic determination of surgical planning data for treating a disease associated with an anatomical target region, e.g., liver cancer. The techniques disclosed herein facilitate incremental and continuous improvement of a reinforcement learning algorithm for surgical planning data for a treatment of a disease associated with an anatomical target region of a patient by leveraging information within each new successful intervention or operation. The reinforcement learning algorithm for surgical planning data can be automatically and continually fine-tuned or tailored to specific requirements or preferences of a clinician or a hospital. The aggregation of information among multiple clinicians and hospitals can be used to build and continually improve a machine learning model configured for determining a reward associated with the reinforcement learning algorithm. The machine learning model can be further improved by taking into account insights from clinicians. Federated learning based on gradient information from local model updates allows secure application in a healthcare system since no patient information is shared with a centralized server.

According to various examples, techniques described herein may overcome the limitations or drawbacks of reference techniques in two ways:
(1) by exploiting existing and newly incoming patient data, e.g., medical imaging and information about the performed thermal ablation procedure like the specific needle position, and comparing the determined surgical planning data against the clinical ones; and
(2) by inquiring clinicians' feedback on the determined surgical planning data for each new intervention.

For example, the acquisition of human feedback on accuracy enables more accurate and clinically relevant surgical planning data. This strategy has already demonstrated remarkable success and has become a favorable strategy in the field of question-answering systems like ChatGPT. Moreover, the information is aggregated globally by means of federated learning. Thus, the reinforcement learning algorithm for surgical planning data can be improved continuously with each new intervention performed at any connected site or hospital.

Independent of the grammatical term usage, individuals with male, female or other gender identities are included within the term.

### List of Reference

[1] Reig M, FornerA, Rimola J, Ferrer-Fàbrega J, Burrel M, Garcia-Criado Á, Kelley RK, Galle PR, Mazzaferro V, Salem R, Sangro B. BCLC strategy for prognosis prediction and treatment recommendation: The 2022 update. Journal of hepatology. 2022 Mar 1;76(3):681-93.
[2] Laimer G, Schullian P, Jaschke N, Putzer D, Eberle G, Alzaga A, Odisio B, Bale R. Minimal ablative margin (MAM) assessment with image fusion: an independent predictor for local tumor progression in hepatocellular carcinoma after stereotactic radiofrequency ablation. European radiology. 2020 May;30:2463-72.
[3] Zhang R, Wu S, Wu W, Gao H, Zhou Z. Computer-assisted needle trajectory planning and mathematical modeling for liver tumor thermal ablation: A review. Mathematical biosciences and engineering. 2019;16(5):4846-72.
[4] Liang L, Cool D, Kakani N, Wang G, Ding H, FensterA. Automatic radiofrequency ablation planning for liver tumors with multiple constraints based on set covering. I EEE transactions on medical imaging. 2019 Nov 4;39(5):1459-71.
[5] Villard C, Soler L, Papier N, Agnus V, Gangi A, Mutter D, Marescaux J. RF-Sim: a treatment planning tool for radiofrequency ablation of hepatic tumors. InProceedings on Seventh International Conference on Information Visualization, 2003. IV 2003. 2003 Jul 18 (pp. 561-566). IEEE.
[6] Baegert C, Villard C, Schreck P, Soler L, Gangi A. Trajectory optimization for the planning of percutaneous radiofrequency ablation of hepatic tumors. Computer Aided Surgery. 2007 Jan 1;12(2):82-90.
[7] Chaitanya K, Audigier C, Balascuta LE, Mansi T. Automatic planning of liver tumor thermal ablation using deep reinforcement learning. In International Conference on Medical Imaging with Deep Learning 2022 Dec 4 (pp. 219-230). PMLR.
[8] Ackermann J, Wieland M, Hoch A, Ganz R, Snedeker JG, Oswald MR, Pollefeys M, Zingg PO, Esfandiari H, Fürnstahl P. A new approach to orthopedic surgery planning using deep reinforcement learning and simulation. InMedical Image Computing and Computer Assisted Intervention-MICCAI 2021: 24th International Conference, Strasbourg, France, September 27-October 1, 2021, Proceedings, Part IV 24 2021 (pp. 540-549). Springer International Publishing.
[9] Rüttgers M, Waldmann M, Vogt K, Ilgner J, Schröder W, Lintermann A. Automated surgery planning for an obstructed nose by combining computational fluid dynamics with reinforcement learning. Computers in biology and medicine. 2024 May 1;173:108383.
[10] Ou Y, Tavakoli M. Sim-to-real surgical robot learning and autonomous planning for internal tissue points manipulation using reinforcement learning. IEEE Robotics and Automation Letters. 2023 Mar 10;8(5):2502-9.
[11] Zhang Q, Li M, Qi X, Hu Y, Sun Y, Yu G. 3D path planning for anterior spinal surgery based on CT images and reinforcement learning. In2018 IEEE international conference on cyborg and bionic systems (CBS) 2018 Oct 25 (pp. 317-321). IEEE.
[12] Mnih V, Kavukcuoglu K, Silver D, Rusu AA, Veness J, Bellemare MG, Graves A, Riedmiller M, Fidjeland AK, Ostrovski G, Petersen S. Human-level control through deep reinforcement learning. nature. 2015 Feb 26;518(7540):529-33.
[13] Dosovitskiy A, Beyer L, Kolesnikov A, Weissenborn D, Zhai X, Unterthiner T, Dehghani M, Minderer M, Heigold G, Gelly S, Uszkoreit J. An image is worth 16x16 words: Transformers for image recognition at scale. arXiv preprint arXiv:2010.11929. 2020 Oct 22.
[14] Wang Z, Bovik AC, Sheikh HR, Simoncelli EP. Image quality assessment: from error visibility to structural similarity. IEEE transactions on image processing. 2004 Apr 13;13(4):600-12.

## Claims

1. A computer-implemented method (2000) for fine-tuning a pre-trained reinforcement learning algorithm, wherein the pre-trained reinforcement learning algorithm is configured to determine surgical planning data for treating a disease associated with an anatomical target region, the method comprising:
- obtaining (2100) one or more instances of the surgical planning data, and obtaining (2200) one or more scores, each of the one or more scores being associated with a quality of the respective instance of the surgical planning data;
- determining (2300), based on each of the one or more instances of the surgical planning data and using a machine learning model, a respective estimated score associated with the quality of the respective instance of the surgical planning data;
- updating (2400) parameter values of the machine learning model based on a comparison between each of the one or more scores and a corresponding estimated score; and,
- fine-tuning (2500) the pre-trained reinforcement learning algorithm using the updated machine learning model for determining a reward associated with the pre-trained reinforcement learning algorithm.

2. The computer-implemented method (2000) of claim 1, wherein said obtaining of the one or more instances of the surgical planning data comprises:
- obtaining (2110) one or more medical images, each of the one or more medical images depicting the anatomical target region;
- determining (2120), based on the one or more medical images, the one or more instances of the surgical planning data using the pre-trained reinforcement learning algorithm.

3. The computer-implemented method (2000) of claim 2, wherein said determining of the one or more instances of the surgical planning data using the pre-trained reinforcement learning algorithm comprises:
- determining (2121), based on the one or more medical images, multiple candidate instances of the surgical planning data using the pre-trained reinforcement learning algorithm;
- selecting (2122), from the multiple candidate instances of the surgical planning data, the one or more instances of the surgical planning data based on a pre-defined criterion.

4. The computer-implemented method (2000) of any one of the preceding claims, further comprising:
- obtaining (2600) a segmented image depicting one or more tissues within the anatomical target region;
wherein said determining of the respective estimated score associated with the quality of the respective instance of the surgical planning data is further based on the segmented image.

5. The computer-implemented method (2000) of any one of the preceding claims, wherein said obtaining of the one or more scores comprises:
- obtaining (2210) a reference surgical planning data as a ground-truth of the surgical planning data;
- determining (2220) the one or more scores based on a comparison between the reference surgical planning data and a corresponding instance of the surgical planning data.

6. The computer-implemented method (2000) of any one of the preceding claims, wherein said fine-tuning of the pre-trained reinforcement learning algorithm comprises:
- obtaining (2510) one or more further medical images, each of the one or more further medical images depicting the anatomical target region;
- processing (2520) the one or more further medical images using an agent module and an environment module of the pre-trained reinforcement learning algorithm together with the updated machine learning model.

7. The computer-implemented method (2000) of any one of the preceding claims, wherein the surgical planning data comprises a thermal ablation planning, which comprises a determination of at least one of the following: an insertion point for an ablation needle, a trajectory for inserting the ablation needle, a safety margin, a target point, an ablation zone, a contour of skin, a contour of a tumor, and one or more ablation configurations.

8. The computer-implemented method (2000) of any one of the preceding claims, further comprising:
- providing (2700), to a central computing device, the updated parameters of the updated machine learning model; and
- upon providing the updated parameters, receiving (2800), from the central computing device, an update of the updated machine learning model and/or an update of the pre-trained reinforcement learning algorithm.

9. The computer-implemented method (2000) of claim 8, wherein the update of the updated machine learning model is performed, by the central computing device, using secure aggregation and/or federated averaging based on the updated parameters of the updated machine learning model and further based on at least one additional update of the parameters of the machine learning model, the at least one additional update of the parameters being received by the central computing device from one or more additional computing devices running the pre-trained reinforcement learning algorithm.

10. The computer-implemented method (2000) of claim 8 or 9, wherein the update of the pre-trained reinforcement learning algorithm is performed, by the central computing device, based on the update of the updated machine learning model for determining the reward associated with the pre-trained reinforcement learning algorithm.

11. The computer-implemented method (2000) of any one of the preceding claims, wherein the machine learning model comprises a convolutional neural network or a transformer-based neural network.

12. A computer-implemented method for determining surgical planning data for a treatment of a disease associated with an anatomical target region of a patient, comprising:
- obtaining one or more medical images, the one or more medical images depicting the anatomical target region of the patient; and
- determining, based on the one or more medical images, the surgical planning data using a reinforcement learning algorithm, fine-tuning of the reinforcement learning algorithm being based on a reward determined based on a pre-trained machine-learning model.

13. The computer-implemented method of claim 12, wherein the reinforcement learning algorithm is fine-tuned by the method (2000) of any one of claims 1 to 11.

14. A computing device (9000), the computing device comprising at least one processor (9020) and the at least one processor (9020) being configured to perform the method (2000) of any one of the preceding claims.

15. A medical imaging equipment (1002a-1002e), the medical imaging equipment (1002a-1002e) comprising the computing device (9000) of claim 14.

## Amended claims

### Amended claims in accordance with Rule 137(2) EPC.

1. A computer-implemented method (2000) for fine-tuning a pre-trained reinforcement learning algorithm, wherein the pre-trained reinforcement learning algorithm is configured to determine surgical planning data for treating a disease associated with an anatomical target region, the method comprising:
- obtaining (2100) one or more instances of the surgical planning data, and obtaining (2200) one or more scores, each of the one or more scores being associated with a quality of the respective instance of the surgical planning data;
- determining (2300), based on each of the one or more instances of the surgical planning data and using a machine learning model, a respective estimated score associated with the quality of the respective instance of the surgical planning data;
- updating (2400) parameter values of the machine learning model based on a comparison between each of the one or more scores and a corresponding estimated score; and,
- fine-tuning (2500) the pre-trained reinforcement learning algorithm using the updated machine learning model for determining a reward associated with the pre-trained reinforcement learning algorithm;
wherein said obtaining of the one or more scores comprises:
- obtaining (2210) a reference surgical planning data as a ground-truth of the surgical planning data;
- determining (2220) the one or more scores based on a comparison between the reference surgical planning data and a corresponding instance of the surgical planning data.

2. The computer-implemented method (2000) of claim 1, wherein said obtaining of the one or more instances of the surgical planning data comprises:
- obtaining (2110) one or more medical images, each of the one or more medical images depicting the anatomical target region;
- determining (2120), based on the one or more medical images, the one or more instances of the surgical planning data using the pre-trained reinforcement learning algorithm.

3. The computer-implemented method (2000) of claim 2, wherein said determining of the one or more instances of the surgical planning data using the pre-trained reinforcement learning algorithm comprises:
- determining (2121), based on the one or more medical images, multiple candidate instances of the surgical planning data using the pre-trained reinforcement learning algorithm;
- selecting (2122), from the multiple candidate instances of the surgical planning data, the one or more instances of the surgical planning data based on a pre-defined criterion.

4. The computer-implemented method (2000) of any one of the preceding claims, further comprising:
- obtaining (2600) a segmented image depicting one or more tissues within the anatomical target region;
wherein said determining of the respective estimated score associated with the quality of the respective instance of the surgical planning data is further based on the segmented image.

5. The computer-implemented method (2000) of any one of the preceding claims, wherein said fine-tuning of the pre-trained reinforcement learning algorithm comprises:
- obtaining (2510) one or more further medical images, each of the one or more further medical images depicting the anatomical target region;
- processing (2520) the one or more further medical images using an agent module and an environment module of the pre-trained reinforcement learning algorithm together with the updated machine learning model.

6. The computer-implemented method (2000) of any one of the preceding claims, wherein the surgical planning data comprises a thermal ablation planning, which comprises a determination of at least one of the following: an insertion point for an ablation needle, a trajectory for inserting the ablation needle, a safety margin, a target point, an ablation zone, a contour of skin, a contour of a tumor, and one or more ablation configurations.

7. The computer-implemented method (2000) of any one of the preceding claims, further comprising:
- providing (2700), to a central computing device, the updated parameters of the updated machine learning model; and
- upon providing the updated parameters, receiving (2800), from the central computing device, an update of the updated machine learning model and/or an update of the pre-trained reinforcement learning algorithm.

8. The computer-implemented method (2000) of claim 7, wherein the update of the updated machine learning model is performed, by the central computing device, using secure aggregation and/or federated averaging based on the updated parameters of the updated machine learning model and further based on at least one additional update of the parameters of the machine learning model, the at least one additional update of the parameters being received by the central computing device from one or more additional computing devices running the pre-trained reinforcement learning algorithm.

9. The computer-implemented method (2000) of claim 7 or 8, wherein the update of the pre-trained reinforcement learning algorithm is performed, by the central computing device, based on the update of the updated machine learning model for determining the reward associated with the pre-trained reinforcement learning algorithm.

10. The computer-implemented method (2000) of any one of the preceding claims, wherein the machine learning model comprises a convolutional neural network or a transformer-based neural network.

11. A computer-implemented method for determining surgical planning data for a treatment of a disease associated with an anatomical target region of a patient, comprising:
- obtaining one or more medical images, the one or more medical images depicting the anatomical target region of the patient; and
- determining, based on the one or more medical images, the surgical planning data using a reinforcement learning algorithm, fine-tuning of the reinforcement learning algorithm being based on a reward determined based on a pre-trained machine-learning model;
wherein the reinforcement learning algorithm is fine-tuned by the method (2000) of claim 1.

12. The computer-implemented method of claim 11, wherein the reinforcement learning algorithm is fine-tuned by the method (2000) of any one of claims 2 to 10.

13. A computing device (9000), the computing device comprising at least one processor (9020) and the at least one processor (9020) being configured to perform the method (2000) of any one of the preceding claims.

14. A medical imaging equipment (1002a-1002e), the medical imaging equipment (1002a-1002e) comprising the computing device (9000) of claim 13.
